# EUROPÄISCHE PATENTSCHRIFT

(11) **EP 1 990 847 B1**
(45) Veröffentlichungstag und Bekanntmachung des Hinweises auf die Patenterteilung: **20.06.2018**
(21) Anmeldenummer: 07009366.1
(22) Anmeldetag: 10.05.2007
(51) Int. Cl.: C07D 207/44, H01L 51/54

(54) **Verwendung von chinoiden Bisimidazolen und deren Derivaten als Dotand zur Dotierung eines organischen halbleitenden Matrixmaterials**
Use of quinoid bisimidazoles and their derivatives as dopant for doping an organic semi-conductor matrix material
Utilisation de bisimidazoles quinoïdes et leurs dérivés en tant que dopant destiné au dopage d'un matériau de matrice organique semi-conducteur

(43) Veröffentlichungstag der Anmeldung: 12.11.2008
(73) Patentinhaber: Novaled GmbH, 01307 Dresden (DE)
(72) Erfinder: Hartmann, Horst, 01326 Dresden (DE); Zeika, Olaf, 01307 Dresden (DE); Ammann, Martin, 01159 Dresden (DE); Dathe, Rene, 09126 Chemnitz (DE)
(74) Vertreter: Bittner, Thomas L.

(56) Entgegenhaltungen:
- WO-A-03/060956
- JP-A- 63 172 275
- JP-A- S63 172 274
- US-A- 6 103 459
- M. MATSCHKE, C. KÄPPLINGER, R. BECKERT: "Bis-4H-imidazoles-tetraazafulvalenes-2,2' -biimidazoles: three variations of one redox system" TETRAHEDRON, ELSEVIER SCIENCE PUBLISHERS, AMSTERDAM, NL, Bd. 62, Nr. 36, 4. September 2006 (2006-09-04), Seiten 8586-8590, XP005577078 Amsterdam, NL; available online 17 July 2006 ISSN: 0040-4020
- CHRISTIANE PETZHOLD: "Beiträge zur Synthese funktioneller 1,4,5,8-Tetraazafulvalene" 10. September 2006 (2006-09-10), DIGITALE BIBLIOTHEK THÜRINGEN , THÜRINGEN, DEUTSCHLAND , XP002455687 * Seite 5, Zeile 1 - Zeile 5; Abbildung 1.1 * * Seite 7, Zeile 6 - Zeile 8 *
- LI J Y ET AL: "Enhancement of green electroluminescence from 2,5-di-p-anisyl-isobenzofuran by double-layer doping strategy" PREPARATION AND CHARACTERIZATION, ELSEVIER SEQUOIA, NL, Bd. 446, Nr. 1, 1. Januar 2004 (2004-01-01), Seiten 111-116, XP004480816 ISSN: 0040-6090
- AKUTAGAWA T ET AL: "Multi electron and proton-transfer system based on 2,2'-biimidazole derivatives" SCIENCE AND TECHNOLOGY OF SYNTHETIC METALS 1994, ICSM '94; INTERNATIONAL CONFERENCE ON 24 - 29 JULY 1994, 24. Juli 1994 (1994-07-24), Seiten 346-346, XP010580195
- U. MAYER, H. BAUMGÄRTEL, H. ZIMMERMANN: "Über 2,3,6,7-Tetraphenyl-1,4,5,8-Tetraazafulval ene" TETRAHEDRON LETTERS, Bd. 7, Nr. 42, 1966, Seiten 5221-5223, XP002455670 Pergamon Press Ltd, Great Britain
- DEDIK S G ET AL: "Tetrahalotetraaazafulvalenes - new strong electron acceptors", CHEMISTRY OF HETEROCYCLIC COMPOUNDS (A TRANSLATION OF KHIMIYAGETEROTSIKLICHESKIKH SOEDINENII), PLENUM PRESS CO., NEW YORK, NY, US, no. 10, 1 October 1989 (1989-10-01), page 1191, XP009104364, ISSN: 0009-3122, DOI: DOI:10.1007/BF00470704

## Beschreibung

Die vorliegende Erfindung betrifft die Verwendung von chinoiden Bisimidazolen und deren Derivaten als p-Dotand zur Dotierung eines organischen halbleitenden Matrixmaterials in elektronischen oder optoelektronischen Bauelementen.

Es ist bekannt, organische Halbleiter durch Dotierung hinsichtlich ihrer elektrischen Eigenschaften, insbesondere ihrer elektrischen Leitfähigkeit, zu verändern, wie dies auch bei anorganischen Halbleitern wie Siliciumhalbleitern der Fall ist. Hierbei wird durch Erzeugung von Ladungsträgern im Matrixmaterial eine Erhöhung der zunächst recht niedrigen Leitfähigkeit sowie je nach Art des verwendeten Dotanden eine Veränderung im Fermi-Niveau des Halbleiters erreicht. Eine Dotierung führt hierbei zu einer Erhöhung der Leitfähigkeit von Ladungstransportschichten, wodurch ohmsche Verluste verringert werden, und zu einem verbesserten Übergang der Ladungsträger zwischen Kontakten und organischer Schicht, wenn sich diese organische Schicht zwischen zwei elektrischen Kontakten entgegengesetzter Polarität befindet. Anorganische Dotanden, wie z.B. Lewis-Säuren als Elektronenakzeptoren (z.B. FeCl_{3;} SbCl₅), sind bei organischen Matrixmaterialien aufgrund ihrer hohen Diffusionskoeffizienten meist nachteilig, da durch sie die Funktion und Stabilität der elektronischen Bauelemente beeinträchtigt wird (D. Oeter, Ch. Ziegler, W. Göpel Synthetic Metals 1993, 61, 147-50; Y. Yamamoto, S. Kanda, S. Kusabayashi, T. Nogaito, K. Ito, H. Mikawa, Bull. Chem. Soc. Jap. 1965, 38, 2015-17; J. Kido et al. Jpn J. Appl. Phys. 2002, 41, 358-60). Überdies weisen letztere Dotanden einen so hohen Dampfdruck auf, dass ein technischer Einsatz in bekannten Prozessierungsanlagen für organisch-elektronische Bauteile sehr fraglich ist. Außerdem sind die Reduktionspotentiale dieser Verbindungen oft zu niedrig, um technisch wirklich interessante Matrixmaterialien zu dotieren. Zusätzlich erschwert das äußerst aggressive chemische Reaktionsverhalten dieser Dotanden eine technische Anwendung.

Aus JP 63-172275 A sind chinoide Bisimidazole zur Verwendung als Ladungsinjektionsschicht oder als Matrixmaterial bekannt, bei denen die Imidazoleinheiten jedoch über eine Brücke miteinander verbunden sind.

Aus Tetrahedron, Band 62, Nr. 36, Seiten 8586-8590, sind ebenfalls chinoide Bisimidazole bekannt. Bei den dort gezeigten Verbindungen weisen die Imidazolkomponenten jedoch stets Aminsubstituenten auf.

Aus C. Petzhold, Dissertation, 20. Juni 2006 "Beiträge zur Synthese funktioneller 1,4,5,8-Tetraazafulvalene" (Universität Jena, Deutschland), sind ebenfalls Verbindungen bekannt, die stets Amin-Substituenten am Imidazolring aufweisen.

In JP 63-172274 wird die Verwendung verschiedener organischer Verbindungen in photoleitenden Filmen in der Elektrophotographie beschrieben. Ein in der Elektrophotographie verwendeter Film kann jedoch keinesfalls dotiert sein, da die geladene Oberfläche aufgrund der erhöhten Leitfähigkeit die Ladung und damit das Bild in diesen Fall nicht halten könnte.

In US 6,103,459 wird in Bezug auf Tetraazafulvalene eine mögliche Verwendung als elektrisch leitfähiges Polymer, ein thermochromes Material und ein molekulares Ladungstransfersalz erwähnt.

Der vorliegenden Erfindung liegt die Aufgabe zugrunde, durch Verwendung von chinoiden Imidazolen und deren Derivaten verbesserte organische halbleitende Matrixmaterialien zur Verwendung in elektronischen oder optoelektronischen Bauelementen bereitzustellen. Insbesondere sollen die verwendeten chinoiden Bisimidazole ausreichend hohe Reduktionspotentiale aufweisen, ohne störende Einflüsse auf das Matrixmaterial sein und eine wirksame Erhöhung der Ladungsträgeranzahi im Matrixmaterial bereitstellen und vergleichsweise einfach handhabbar sein, im Sinne ihrer Verwendung in der Prozessierung elektronischer Bauteile.

Weitere Aufgaben der Erfindung liegen in der Bereitstellung von organischen halbleitenden Materialien und von elektronischen Bauelementen oder optoelektronischen Bauelementen, in denen die offenbarten Verbindungen als p-Dotanden verwendet werden können.

Die erste Aufgabe wird gelöst durch die Verwendung von chinoiden Bisimidazolen und Derivaten derselben als p-Dotand zur Dotierung eines organischen halbleitenden Matrixmaterials in elektronischen oder optoelektronischen Bauelementen, wobei die chinoiden Bisimidazole durch die folgende Formel dargestellt werden: 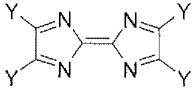 wobei jedes Y unabhängig ausgewählt wird aus der Gruppe bestehend aus Wasser-stoff, substituierten und unsubstituierten Arylen, substituierten und unsubstituierten Heteroarylen, substituierten und unsubstituierten konjugierten Kohlenwasserstoffket-ten mit alternierenden C-C-Einfach- und Doppelbindungen, Halogen, Cyano, Pseudo-halogen, Nitro, Fluor- und Perfluoralkylen, Carbonsäure und deren Derivaten, Sulfon-säuren und deren Derivaten, oder wobei zwei benachbarte Y Bestandteil eines an den Imidazolring annelierten aromatischen Ringsystems sind.

Unter Arylen und Heteroarylen sollen auch Oligo- und Polyaryle bzw. -Heteroaryle verstanden werden.

Ebenfalls ist optional vorgesehen, dass das an den Imidazolring annelierte aromatische Ringsystem substituiert ist.

Erfindungsgemäß ist ebenfalls ein organisches halbleitendes Material, enthaltend zumindest eine organische Matrixverbindung und einen p-Dotanden, wobei als p-Dotand zumindest eine Verbindung der folgenden Formel verwendet wird: 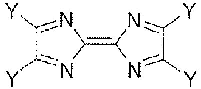 wobei jedes Y unabhängig ausgewählt wird aus der Gruppe bestehend aus Wasser-stoff, substituierten und unsubstituierten Arylen, substituierten und unsubstituierten Heteroarylen, substituierten und unsubstituierten konjugierten Kohlenwasserstoffket-ten mit alternierenden C-C-Einfach- und Doppelbindungen, Halogen, Cyano, Pseudo-halogen, Nitro, Fluor- und Perfluoralkylen, Carbonsäure und deren Derivaten, Sulfon-säuren und deren Derivaten, oder wobei zwei benachbarte Y Bestandteil eines an den Imidazolring annelierten aromatischen Ringsystems sind.

Dabei ist bevorzugt, dass das molare Dotierungsverhältnis von Dotand zu Matrixmolekül bzw. das Dotierungsverhältnis von Dotand zu monomeren Einheiten eines polymeren Matrixmoleküls zwischen 20:1 und 1:100.000, bevorzugt 10:1 und 1:1000, besonders bevorzugt 1:1 und 1:100, beträgt.

Erfindungsgemäß ist ebenfalls ein elektronisches oder optoelektronisches Bauelement mit einem elektronisch funktionell wirksamen Bereich, wobei der elektronisch funktionell wirksame Bereich ein organisches halbleitendes Matrixmaterial aufweist, welches mit zumindest einem p-Dotanden zur Veränderung der elektronischen Eigenschaften des halbleitenden Matrixmaterials unter Verwendung zumindest einer Verbindung der folgenden Formel dotiert ist: 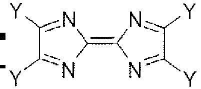 wobei jedes Y unabhängig ausgewählt wird aus der Gruppe bestehend aus Wasserstoff, substituierten und unsubstituierten Arylen, substituierten und unsubstituierten Heteroarylen, substituierten und unsubstituierten konjugierten Kohlenwasserstoffketten mit alternierenden C-C-Einfach- und Doppelbindungen, Halogen, Cyano, Pseudohalogen, Nitro, Fluor- und Perfluoralkylen, Carbonsäure und deren Derivaten, Sulfonsäuren und deren Derivaten, oder wobei zwei benachbarte Y Bestandteil eines an den Imidazolring annelierten aromatischen Ringsystems sind und optional das an den Imidazolring annelierte aromatische Ringsystem substituiert ist.

Schließlich wird vorgeschlagen, dass das elektronische oder optoelektroische Bauelement in Form einer organischen licht-emittierenden Diode, einer photovoltaischen Zelle, einer organischen Solarzelle, einer organischen Diode oder eines organischen Feldeffekttransistors ist.

Schließlich ist erfindungsgemäß auch die Verwendung des chinoiden Bisimidazoles mit obiger Strukturformel als p-Dotand zur Dotierung eines organischen halbleitenden Matrixmaterials in einer p-dotierten Schicht in einem elektronischen oder optoelektronischen Bauelementen vorgesehen, wobei in dem elektronischen oder optoelektronisch Bauelement die genannte p-dotierte Schicht auf Basis eines organischen Matrixmaterials in folgenden Schichtstrukturen vorliegt: p-i-n: mit p-Dotand p-dotierter Halbleiter - intrinisisch halbleitfähige Schicht - n-dotierter Halbleiter; n-i-p: n-dotierter Halbleiter - intrisisch halbleitfähige Schicht - p-dotierter Halbleiter; oder p-n oder n-p-Übergänge.

Überraschenderweise wurde festgestellt, dass bei erfindungsgemäßer Verwendung der offenbarten chinoiden Bisimidazole ein wesentlich stärkerer und/oder stabilerer p-Dotand als bei bisher bekannten Donorverbindungen vorliegt.

Insbesondere wird die Leitfähigkeit von Ladungstransportschichten bei erfindungsgemäßer Verwendung wesentlich erhöht und/oder der Übergang der Ladungsträger zwischen den Kontakten und organischer Schicht bei Anwendungen als elektronisches Bauelement wesentlich verbessert. Ohne durch diese Vorstellung eingeschränkt zu sein, wird davon ausgegangen, dass bei erfindungsgemäßer Verwendung der chinoiden Bisimidazole in einer dotierten Schicht CT-Komplexe gebildet werden, insbesondere durch den Transfer von mindestens einem Elektron vom jeweiligen umgebenden Matrixmaterial an den Dotanden. Hierbei werden Kationen des Matrixmaterials gebildet, die auf dem Matrixmaterial beweglich sind. Auf diese Weise gewinnt das Matrixmaterial eine Leitfähigkeit, die gegenüber der Leitfähigkeit des undotierten Matrixmaterials erhöht ist. Leitfähigkeiten von undotierten Matrixmaterialien sind in der Regel < 10⁻⁸ S/cm, insbesondere häufig < 10⁻¹⁰ S/cm. Es ist dabei darauf zu achten, dass die Matrixmaterialien eine genügend hohe Reinheit aufweisen. Solche Reinheiten sind mit herkömmlichen Methoden, zum Beispiel Gradientensublimation, zu erreichen. Durch Dotierung lässt sich die Leitfähigkeit solcher Matrixmaterialien auf größer 10⁻⁸ S/cm, häufig > 10⁻⁵ S/cm erhöhen. Dies gilt insbesondere für Matrixmaterialien, die ein Oxidationspotential von größer als -0.5 V vs. Fc/Fc⁺, bevorzugt größer 0 V vs. Fc/Fc⁺, insbesondere größer +0.2 V vs. Fc/Fc⁺ aufweisen. Die Angabe Fc/Fc⁺ bezieht sich auf das Redoxpaar Ferrocen/ Ferrocenium, das als Referenz in einer elektrochemischen Potentialbestimmung, zum Beispiel der Cyclovoltammetrie, eingesetzt wird.

Die in dieser Erfindung beschriebenen Dotanden weisen eine überraschend hohe Stabilität in Bezug auf ihre Reaktivität mit der Atmosphäre auf.

Die als Dotanden eingesetzten Bisimidazole können neben der gezeichneten chinoiden Form auch ausgeprägten biradikalischen Charakter oder zwitterionischen Charakter in ihrer stabilsten Struktur aufweisen (A. Kikuchi, H. Ito, J. Abe, J. Phys. Chem. B 2005, 109, 19448-19453; A. Kikuchi, F. Iwahori, J. Abe, J. Am. Chem. Soc. 2004, 126, 6526-6527; T. Suzuki, et al., Tetrahedron Lett. 2003, 44, 7881-7884; K. Okada et al., Chem. Lett. 1998, 891-892; M. Kozaki, A. Isoyama, K. Okada, Tetrahedron Lett. 2006, 47, 5375-5378; S. Yoshiko et al., Nippon Kagaku Kaishi, 1972, 1, 100-3). Derivate der hier beschriebenen chinoiden Bisimidazole wurden bereits verwendet als Fluoreszenzfarbstoffe (R. Beckert et al., DE 10261662), als Photosensitizer in der photodynamischen Therapie (B. Bilbao et al., WO 2003104237), des weiteren als Precursoren für die chemische Vakuumdeposition von Materialien für organische elektronische Bauteile (D. Bruce, WO 2000053613) und für elektrophotographische Anwendungen als Materialien in photoleitfähigen Schichten (F. Katsunori, JP 63172274).

### Darstellung der chinoiden Bisimidazole und deren Derivate

Die beschriebenen chinoiden Bisimidazole lassen sich nach bekannten Verfahren synthetisieren. Die Darstellung geht von der Bereitstellung benzoider Bisimidazole aus, die in ihre Anionen überführt werden und durch geeignete Oxidationsmittel in die chinoiden Bisimidazole überführt werden, nach folgendem allgemeinen Schema:

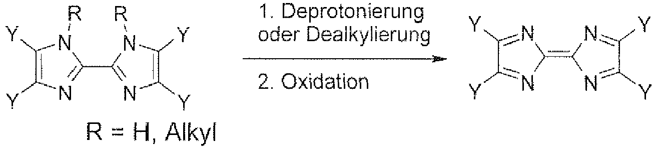

Hierbei kann die Oxidation auch ohne vorherige Deprotonierung direkt aus der Form der benzoiden Bisimidazole erfolgen oder aus einer Vorstufe mit Substitution der N-Atome (R = beliebiger verzweigter oder unverzeigter Alkylrest oder substituierter Alkylrest). Beispiele für die Darstellung der dafür erforderlichen Ausgangstoffe, der Aryl-verbrückten benzoiden Bisimidazole (X = Aryl oder Heteroaryl), sind ausführlich in der Literatur beschrieben und basieren z.B. auf der Kondensation von Diketonen mit Aryl- oder Heteroarylcarbodialdehyden (M. Weiss, J. Am. Chem. Soc. 1952, 74, 5193-5195; U. Mayer, H. Baumgärtel, H. Zimmermann, Tetrahedron Lett. 1966, 42, 5221-5223; P. Schneiders, J. Heinze, H. Baumgärtel, Chem. Ber. 1973, 106, 2415-2417; M. Kozaki, A. Isoyama, K. Akita, K. Okada, Org. Lett. 2005, 7, 115-118; L.-N. Ji, X.-Y. Li et al., J. Chem Soc., Dalton Trans. 2001, 1920-1926; F. C. Krebs et al., Tetrahedron Lett. 2001, 42, 6753-6757). Auch unsymmetrische Derivate Aryl-verbrückter benzoider Bisimidazole sind so zugänglich (M. Kimura et al., ITE Letters on Batteries, New Technologies and Medicine 2002, 3, 30-34). Eine andere Art der Darstellung von Bisimidazolen ist die Kondensation von Diaminen mit Carbodialdchyden unter anschließender oxidativer Cyclisierung des gebildeten Diimins (R. Leyden et al., J. Org. Chem. 1983, 48, 727-731; P. Gogoi, D. Konwar, Tetrahedron Lett. 2006, 47, 79-82). Auch können Bisimidazole durch Kondensation von Diaminen mit Oxalsäurederivaten oder mit entsprechenden Dicarbonsäuren dargestellt werden (E. S. Lane, J. Chem Soc. 1953, 2238-2240; H. Suschitzky, J. Heterocyclic Chem. 1999, 36, 1001-1012; J. Hill, J. Org. Chem. 1963, 28, 1931-2; P. C. Vyas, C. Oza, A. K. Goyal, Chem. Industry 1980, 287-288) oder durch Kondensation von Diketonen mit Glyoxal (F. Japp, E. Cleminshaw, J. Chem. Soc. Trans. 1887, 51, 552-557) oder durch Umsetzung von Phenylendiaminen mit Hexachloraceton (M. C. Rezende, E. L. Dall'Oglio, C. Zucco, Syn. Comm. 2001, 31, 607-613; M. Rezende, E. Dall'Oglio C. Zucco, Tetrahedron Lett. 1996, 37, 5265-5268;). Synthesen von Bisimidazolen können auch erfolgen durch katalytische Dimerisierung von Imidazolen oder Benzimidazolen oder deren N-alkylierten Derivaten (T. Sekine et al., Chem. Pharm. Bull. 1989, 37, 1987-1989) oder durch metallorganische, in der Regel Kupfer-vermittelte Verknüpfung von 2-metallierten Imidazolen oder Benzimidazolen (S. B. Park, H. Alper, Organic Lett. 2003, 5, 3209-3212; F. Bonati, A. Burini, B. R. Pietroni, J. Organomet. Chem. 1989, 375, 147-160) oder durch Ringverknüpfung zwischen einem Imidazol und einem 2-Diazoimidazol (P. G. Rasmussen et al., J. Am. Chem. Soc. 1982, 104, 6155-6156).

Die direkte Oxidation benzoider Bisimidazole zu den eigentlichen p-Dotanden den chinoiden Bisimidazolen, durch gängige Oxidationsmittel wie Mangandioxid oder Blei(IV)acetat ist bekannt (U. Mayer, H. Baumgärtel, H. Zimmermann, Tetrahedron Lett. 1966, 42, 5221-5223; H. Suschitzky, J. Heterocyclic Chem. 1999, 36, 1001-1012; J. Hill, J. Org. Chem. 1963, 28, 1931-1932) und kann darüber hinaus erfolgen direkt aus den benzoiden Bisimidazolen in einem 2-Phasengemisch der organischen Lösung des benzoiden Precursors mit dem Oxidationsmittel, z.B. Kaliumferricyanid, in einer basischen wässrigen Lösung (M. Kozaki, A. Isoyama, K. Akita, K. Okada, Org. Lett. 2005, 7, 115-118; K. Okada et al., Chem. Lett. 1998, 891-892; Cherkashin M. I. et al. Izv. Akad. Nauk SSSR, Seriya Khim. 1982, 2, 376-377) oder durch Oxidation des Silbersalzes oder des Natriumsalzes des benzoiden Bisimidazoles mit Brom in organischen Lösungsmitteln (U. Mayer, H. Baumgärtel, H. Zimmermann, Angew. Chem. 1966, 78, 303; S. Dedik et al. Khimiya Get. Soed. 1989, 10, 1421).

Auch können die als p-Dotand verwendeten chinoiden Bisimidazole durch photochemische Anregung geeigneter Vorstufen, beispielsweise unter UV Bestrahlung, erzeugt werden (Y. Sakaino, J. Org. Chem. 1979, 44, 1241-1244). Diese Vorstufen sind in der Regel Alkoxyaddukte der chinoiden Bisimidazole, welche wiederum durch den Zusatz von Alkoholen in der basenkatalysierten Oxidation der benzoiden Bisimidazole zu chinoiden Bisimidazolen zugänglich sind.

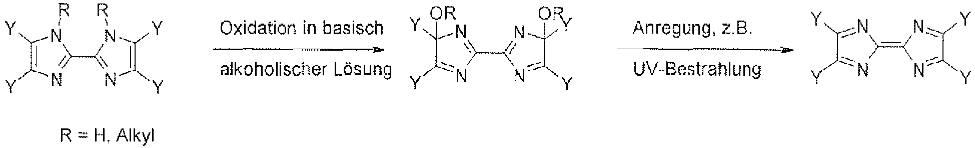

### Ausführungsbeispiel zur Synthese eines chinoiden Bisimidazols

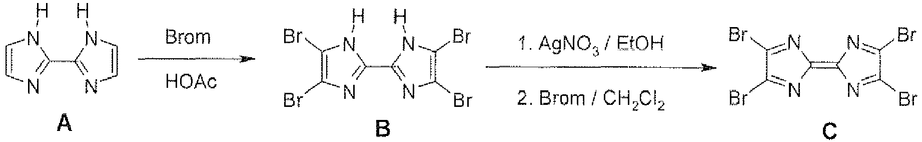

Das Tetrabrombisimidazol B wurde durch Bromierung von käuflichem Bisimidazol A in Abwandlung einer bekannten Vorschrift (K. Lehmstaedt, H. Rolker, Ber. Dt. Chem. Ges. B 1943, 879-891) dargestellt: 520 mg (3.88 mmol) Bisimidazol und 2,13 g (15,7 mmol) Natriumacetat-Trihydrat werden in 10 mL Eisessig vorgelegt, eine Lösung von 2,48 g (15,5 mmol) Brom in 10 mL Eisessig zugetropft. Nach beendeter Zugabe wird für 2 h auf 100°C erhitzt. Der Ansatz wird zur Trockene eingeengt und mit verdünnter Salzsäure ausgekocht, heiß abgesaugt, mit verdünnter HCl und Wasser gewaschen und getrocknet. Ausbeute 1.11 g B (64% d.Th.). HPLC-Untersuchung des Produkts ergab eine Reinheit von 90%, DI-MS (EI) m/z = 450, das Isotopenmuster korrespondiert mit 4 Bromsubstituenten.

Die Darstellung des chinoiden Dotanden C erfolgte in Abwandlung einer bekannten Vorschrift (S. Dedik et al. Khimiya Gel. Soed. 1989, 10, 1421) durch Erhitzen von 565 mg (1.26 mmol) Tetrabromdiimidazol B mit 428 mg (2,52 mmol) Silbernitrat in 40 ml absolutem EtOH für 1 h unter Rückfluss und Absaugen des gebildeten Produkts. Dieses Produkt wurde in 20 mL absolutem Dichlormethan suspendiert, auf 0°C gekühlt und mit 12,8 mL 0,1 M Brom in Dichlormethan versetzt. Der Ansatz wurde 15 min unter Kühlung und 30 min bei Raumtemperatur gerührt, von den Salzen abgesaugt, mit Dichlormethan gewaschen und die vereinten Filtrate eingeengt. Rohausbeute 418 mg (74% d.Th.). DI-MS (EI): m/z = 448. Vor seiner Testung als Dotand wurde das Rohprodukt bei 160-170°C und ca. 10⁻⁴ mbar einer Gradientensublimation unterzogen. Das sublimierte Produkt ergab in einer cyclovoltammetrischen Untersuchung ein erstes Reduktionspotential von -0.24 V gegenüber Fc/Fc⁺.

### Durchführung der Dotierung

Als p-dotierbare Matrixmaterialien können unter anderem Phthalocyaninkomplexe, beispielsweise des Zn (ZnPc), Cu (CuPc), Ni (NiPc) oder anderer Metalle, wobei der Phthalocyaninligand auch substituiert sein kann, eingesetzt werden. Auch andere Metallkomplexe von Naphthocyaninen und Porphyrinen können gegebenenfalls eingesetzt werden. Weiterhin können als Matrixmaterial auch arylierte oder heteroarylierte Amine bzw. Benzidinderivate eingesetzt werden, die substituiert oder unsubstituiert sein können, beispielsweise TPD, α-NPD, TDATA, insbesondere auch Spiroverknüpfte Arylamine wie z.B. Spiro-TTB. Insbesondere können α-NPD und Spiro-TTB als Matrixmaterial eingesetzt werden.

| | | |
|---|---|---|
| 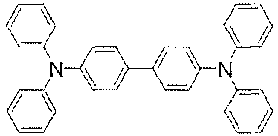 | 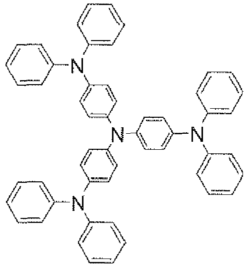 | 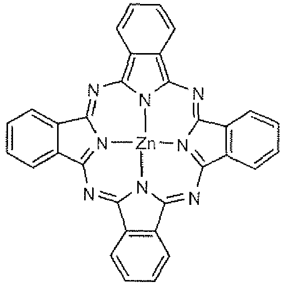 |
| TPD | TDATA | ZnPc |
| 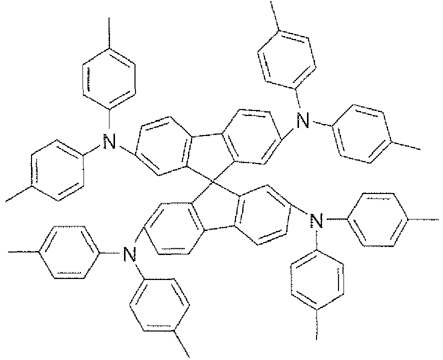 | | 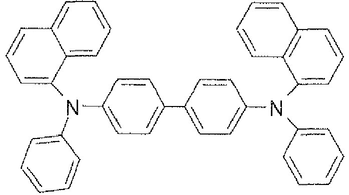 |
| spiro-TTB | | α-NPD |

Als Matrixmaterial können neben polyaromatischen Kohlenwasserstoffen auch Heteroaromaten wie insbesondere Imidazolderivate, Thiophen, Thiazolderivate, Heterotriphenylene aber auch andere eingesetzt werden, gegebenenfalls auch dimere, oligomere bzw. polymere Heteroaromaten. Die Heteroaromaten sind vorzugsweise substituiert, insbesondere Aryl-substituiert, beispielsweise Phenyl- oder Naphthyl-substituiert. Sie können auch als Spiroverbindungen vorliegen.

Es versteht sich, dass die genannten Matrixmaterialien auch untereinander oder mit anderen Materialien gemischt im Rahmen der Erfindung einsetzbar sind. Es versteht sich, dass auch geeignete andere organische Matrixmaterialien verwendet werden können, die halbleitende Eigenschaften aufweisen.

### Dotierungskonzentration

Vorzugsweise liegt der p-Dotand in einer Dotierungskonzentration von ≤ 1:1 zu dem Matrixmolekül bzw. der monomeren Einheit eines polymeren Matrixmoleküls vor, vorzugsweise in einer Dotierungskonzentration von 1:2 oder kleiner, besonders bevorzugt von 1:5 oder kleiner oder 1:10 oder kleiner. Die Dotierungskonzentration kann sich in dem Bereich von 1:1 bis 1:100.000, insbesondere in dem Bereich von 1:5 bis 10.000 oder 1:10 bis 1.000 betragen, beispielsweise in dem Bereich von 1:10 bis 1:100 oder 1:25 bis 1:50, ohne hierauf beschränkt zu sein.

### Durchführung der Dotierung

Die p-Dotierung des jeweiligen Matrixmaterials mit den erfindungsgemäß zu verwendenden Verbindungen kann durch eines oder eine Kombination der folgenden Verfahren erfolgen:
- Mischverdampfung im Vakuum mit einer Quelle für das Matrixmaterial und einer für den p-Dotanden.
- Sequentielles Deponieren des Matrixmaterials und des p-Dotanden auf einem Substrat mit anschließender Eindiffusion des Dotanden, insbesondere durch thermische Behandlung.
- Dotierung einer Matrixschicht durch eine Lösung von p-Dotanden mit anschließendem Verdampfen des Lösungsmittels, insbesondere durch thermische Behandlung.
- Oberflächendotierung einer Matrixmaterialschicht durch eine oberflächlich aufgebrachte Schicht von p-Dotanden.
- Herstellung einer Lösung von Matrixmolekülen und p-Dotanden und anschließende Herstellung einer Schicht aus dieser Lösung mittels konventioneller Methoden wie beispielsweise Verdampfen des Lösungsmittels oder Aufschleudern

Es versteht sich, dass auch andere geeignete Verfahren zur Durchführung der p-Dotierung eingesetzt werden können. Auf diese Weise können somit p-dotierte Schichten von organischen Halbleitern hergestellt werden, die vielfältig einsetzbar sind.

### Halbleitende Schicht

Mittels der erfindungsgemäß verwendeten elektronenarmen chinoiden Bisimidazole können halbleitende Schichten erzeugt werden, die gegebenenfalls eher linienförmig ausgebildet sind, wie z.B. als Leitfähigkeitspfade, Kontakte oder dergleichen. Die chinoiden Bisimidazole können hierbei als p-Dotanden zusammen mit einer anderen Verbindung, die als Matrixmaterial fungieren kann, eingesetzt werden, wobei das Dotierungsverhältnis 1 : 1 oder kleiner sein kann. Der verwendete p-Dotand kann zu der jeweils anderen Verbindung bzw. Komponente aber auch in höheren Anteilen vorliegen, so dass das Verhältnis Dotand : Verbindung im Verhältnis > 1 : 1 liegen kann, beispielsweise im Verhältnis ≥ 2 : 1, ≥ 5 : 1, ≥ 10 : 1 oder ≥ 20 : 1 oder höher. Die jeweils andere Komponente kann eine solche sein, wie sie als Matrixmaterial Falle der Herstellung p-dotierter Schichten eingesetzt werden kann, ohne hierauf beschränkt zu sein.

Der einen p-Dotanden enthaltende Bereich kann insbesondere mit einem organischen halbleitenden Material und/oder einem anorganischen halbleitenden Material elektrisch stromleitend kontaktiert sein, beispielsweise auf einem derartigen Substrat angeordnet sein.

Vorzugsweise werden die genannten elektronenarmen chinoiden Bisimidazole erfindungsgemäß als p-Dotanden eingesetzt, z.B. in einem Verhältnis ≤ 1 : 1 oder ≤ 1 : 2. Mittels der erfindungsgemäß als p-Dotanden eingesetzten elektronenarmen Verbindungen können beispielsweise bei der Verwendung von ZnPc, Spiro-TTB oder α-NPD als Matrix halbleitende Schichten mit Leitfähigkeiten bei Raumtemperatur in dem Bereich von 10⁻⁵ S/cm oder höher erzielt werden. Bei der Verwendung von Phthalocyanin-Zink (ZnPc) als Matrix wurde im Ausführungsbeispiel eine Leitfähigkeit von höher 10⁻⁵ S/cm erzielt. Die Leitfähigkeit von undotiertem Phthalocyanin-Zink beträgt hingegen maximal 10⁻¹⁰ S/cm.

Es versteht sich, dass die Schicht oder das Gebilde mit den p-Dotanden jeweils ein oder mehrere verschiedene derartige elektronenarme chinoide Bisimidazole enthalten kann.

### Elektronisches Bauelement

Unter Verwendung der beschriebenen Verbindungen zur Herstellung p-dotierter organischer halbleitender Materialien, die insbesondere in Form von Schichten oder elektrischen Leitungspfaden angeordnet sein können, können eine Vielzahl elektronischer Bauelemente oder diese enthaltende Einrichtungen mit einer p-dotierten organischen Halbleiterschicht hergestellt werden. Im Sinne der Erfindung werden von dem Begriff "elektronische Bauelemente" auch optoelektronische Bauelemente mit umfasst. Durch die beschriebenen Verbindungen können die elektronischen Eigenschaften eines elektronisch funktionell wirksamen Bereichs des Bauelementes, wie dessen elektrische Leitfähigkeit, lichtemittierende Eigenschaften oder dergleichen, vorteilhaft verändert werden. So kann die Leitfähigkeit der p-dotierten Schichten verbessert und/oder die Verbesserung der Ladungsträgerinjektion von Kontakten in die dotierte Schicht erreicht werden.

Die Erfindung umfasst insbesondere organische lichtemittierende Dioden (OLED), organische Solarzellen, Feldeffekt-Transistoren organische Dioden, insbesondere solche mit hohem Gleichrichtungsverhältnis wie 10³-10⁷, vorzugsweise 10⁴-10⁷ oder 10⁵-10⁷, und organische Feldeffekttransistoren, die mittels der elektronenarmen chinoiden Bisimdazole als p-Dotand werden können.

In dem elektronischen Bauelement kann eine p-dotierte Schicht auf Basis eines organischen Matrixmaterials beispielsweise in folgenden Schichtstrukturen vorliegen, wobei vorzugsweise die Basismaterialien oder Matrixmaterialien der einzelnen Schichten jeweils organisch sind:
p-i-n: p-dotierter Halbleiter - intrinsisch halbleitfähige Schicht - n-dotierter Halbleiter,
n-i-p: n-dotierter Halbleiter - intrinsisch halbleitfähige Schicht - p-dotierter Halbleiter.

Die Kontaktmaterialien sind hier löcherinjizierend, wobei p-seitig beispielsweise eine Schicht oder ein Kontakt aus ITO oder Au vorgesehen sein kann, oder elektroneninjizierend, wobei n-seitig eine Schicht oder ein Kontakt aus ITO, Al oder Ag vorgesehen sein kann.

In obigen Strukturen kann im Bedarfsfall auch die i-Schicht ausgelassen werden, wodurch Schichtenabfolgen mit p-n oder n-p-Übergängen erhalten werden können.

Die Verwendung der beschriebenen Verbindungen ist jedoch auf die oben genannten Ausführungsbeispiele nicht beschränkt, da die Schichtstrukturen durch Einführung zusätzlicher geeigneter Schichten ergänzt bzw. modifiziert werden. Insbesondere können jeweils OLEDs mit derartigen Schichtabfolgen, insbesondere mit p-i-n- oder mit einer dazu inversen Struktur, mit den beschriebenen Verbindungen aufgebaut werden.

Mit Hilfe der beschriebenen p-Dotanden können insbesondere organische Dioden vom Typ Metall - intrinsischer Halbleiter - p-dotierter Halbleiter (m-i-n) oder auch gegebenenfalls vom p-i-n-Typ hergestellt werden, beispielsweise auf der Basis von Phthalocyaninzink. Diese Dioden zeigen ein Rektifizierungsverhältnis (Gleichrichtungsverhältnis, bezogen auf den Stromfluss in Durchgangsrichtung gegenüber dem Stromfluss in Sperrrichtung des Bauteils) von 10⁵ und höher. Ferner können unter Verwendung der erfindungsgemäßen p-Dotanden elektronische Bauelemente mit p-n-Übergängen erzeugt werden, wobei für die p- und die n-dotierte Seite jeweils dasselbe Halbleitermaterial verwendet wird (Homo-p-n-Übergang), und wobei für das p-dotierte Halbleitermaterial ein beschriebenes elektronenarmes chinoides Bisimidazol eingesetzt wird.

Weitere Aufgaben und Vorteile der vorliegenden Erfindung werden nun anschaulich anhand des folgenden Beispiels beschrieben, das lediglich veranschaulichend und nicht als den Umfang der Erfindung begrenzend zu betrachten ist.

### Ausführungsbeispiel zur Dotierung mittels eines chinoiden Bisimdazols

Es wird ein elektronenarmes chinoides Bisimidazol als p-Dotand bereitgestellt, gereinigt durch mindestens einmalige Gradientensublimation im Hochvakuum.

Der vorgelegte p-Dotand wird gleichzeitig mit dem Matrixmaterial verdampft. Gemäß dem Ausführungsbeispiel ist das Matrixmaterial jeweils Phthalocyanin-Zink oder Spiro-TTB. Der p-Dotand und das Matrixmaterial können derart verdampft werden, dass die auf einem Substrat in einer Vakuumverdampfungsanlage niedergeschlagene Schicht ein molares Dotierungsverhältnis von p-Dotand zu Matrixmaterial von 1:10 aufweist.

Die jeweils mit dem p-Dotanden dotierte Schicht des organischen Halbleitermaterials ist auf einer ITO-Schicht (Indiumzinnoxid) aufgebracht, welche auf einem Glassubstrat angeordnet ist. Nach Aufbringung der p-dotierten organischen Halbleiterschicht wird eine Metallkathode aufgebracht, beispielsweise durch Aufdampfung eines geeigneten Metalls, um eine organische Leuchtdiode herzustellen. Es versteht sich, dass die organische Leuchtdiode auch einen sogenannten invertierten Schichtaufbau haben kann, wobei die Schichtenabfolge ist: Glassubstrat - Metallkathode - p-dotierte organische Schicht - transparente leitende Deckschicht (beispielsweise ITO). Es versteht sich, dass je nach Anwendungsfall zwischen den einzelnen genannten Schichten weitere Schichten vorgesehen sein können.

### Beispiel

Das chinoide Bisimidazolderivat C wurde zur Dotierung von ZnPc und spiro-TTB als Matrixmaterial verwandt. Dotierte Schichten mit einem Dotierungsverhältnis Dotand : Matrixmaterial von 1 : 10 wurden durch Mischverdampfung von Matrix und Dotand hergestellt. Die gemessenen Leitfähigkeiten betrugen 7.9x10⁻⁵ S/cm in ZnPc und 4.6x10⁻⁷ S/cm in spiro-TTB.

## Patentansprüche

1. Verwendung von chinoiden Bisimidazolen und Derivaten derselben als p-Dotand zur Dotierung eines organischen halbleitenden Matrixmaterials in elektronischen oder optoelektronischen Bauelementen, wobei die chinoiden Bisimidazole durch die folgende Formel dargestellt werden: 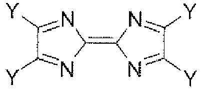 wobei jedes Y unabhängig ausgewählt wird aus der Gruppe bestehend aus Wasserstoff, substituierten und unsubstituierten Arylen, substituierten und unsubstituierten Heteroarylen, substituierten und unsubstituierten konjugierten Kohlenwasserstoffketten mit alternierenden C-C-Einfach- und Doppelbindungen, Halogen, Cyano, Pseudohalogen, Nitro, Fluor- und Perfluoralkylen, Carbonsäure und deren Derivaten, Sulfonsäuren und deren Derivaten, oder wobei zwei benachbarte Y Bestandteil eines an den Imidazolring annelierten aromatischen Ringsystems sind.

2. Verwendung nach Anspruch 1, **dadurch gekennzeichnet, dass** das an den Imidazolring annelierte aromatische Ringsystem substituiert ist.

3. Organisches halbleitendes Material, enthaltend zumindest eine organische Matrixverbindung und einen p-Dotanden, **dadurch gekennzeichnet, dass** als p-Dotand zumindest eine Verbindung der folgenden Formel verwendet wird: 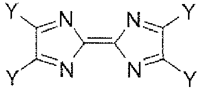 wobei jedes Y unabhängig ausgewählt wird aus der Gruppe bestehend aus Wasserstoff, substituierten und unsubstituierten Arylen, substituierten und unsubstituierten Heteroarylen, substituierten und unsubstituierten konjugierten Kohlenwasserstoffketten mit alternierenden C-C-Einfach- und Doppelbindungen, Halogen, Cyano, Pseudohalogen, Nitro, Fluor- und Perfluoralkylen, Carbonsäure und deren Derivaten, Sulfonsäuren und deren Derivaten, oder wobei zwei benachbarte Y Bestandteil eines an den Imidazolring annelierten aromatischen Ringsystems sind.

4. Organisches halbleitendes Material nach Anspruch 3, **dadurch gekennzeichnet, dass** das an den Imidazolring annelierte aromatische Ringsystem substituiert ist.

5. Organisches halbleitendes Material nach Anspruch 3 oder 4, **dadurch gekennzeichnet, dass** das molare Dotierungsverhältnis von p-Dotand zu Matrixmolekül bzw. das Dotierungsverhältnis von p-Dotand zu monomeren Einheiten eines polymeren Matrixmoleküls zwischen 20:1 und 1:100.000, bevorzugt 10:1 und 1:1000, besonders bevorzugt 1:1 und 1:100, beträgt.

6. Elektronisches oder optoelektronisches Bauelement mit einem elektronisch funktionell wirksamen Bereich, **dadurch gekennzeichnet, dass** der elektronisch funktionell wirksame Bereich ein organisches halbleitendes Matrixmaterial aufweist, welches mit zumindest einem p-Dotanden zur Veränderung der elektronischen Eigenschaften des halbleitenden Matrixmaterials unter Verwendung zumindest einer Verbindung der folgenden Formel dotiert ist: 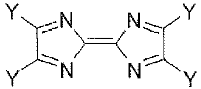 wobei jedes Y unabhängig ausgewählt wird aus der Gruppe bestehend aus Wasserstoff, substituierten und unsubstituierten Arylen, substituierten und unsubstituierten Heteroarylen, substituierten und unsubstituierten konjugierten Kohlenwasserstoffketten mit alternierenden C-C-Einfach- und Doppelbindungen, Halogen, Cyano, Pseudohalogen, Nitro, Fluor- und Perfluoralkylen, Carbonsäure und deren Derivaten, Sulfonsäuren und deren Derivaten, oder wobei zwei benachbarte Y Bestandteil eines an den Imidazolring annelierten aromatischen Ringsystems sind.

7. Elektronisches oder optoelektronisches Bauelement nach Anspruch 6, **dadurch gekennzeichnet, dass** das an den Imidazolring annelierte aromatische Ringsystem substituiert ist.

8. Elektronisches oder optoelektronisches Bauelement nach Anspruch 6 oder 7 in Form einer organischen Licht-emittierenden Diode, einer photovoltaischen Zelle, einer organischen Solarzelle, einer organischen Diode oder eines organischen Feldeffekttransistors.

9. Verwendung von chinoiden Bisimidazolen und Derivaten derselben nach Anspruch 1 als p-Dotand in einer Schicht, wobei in dem elektronischen oder optoelektronischen Bauelement diese p-dotierte Schicht auf Basis eines organischen Matrixmaterials in folgenden Schichtstrukturen vorliegt:
p-i-n: mit p-Dotand p-dotierter Halbleiter - intrinisisch halbleitfähige Schicht - n-dotierter Halbleiter;
n-i-p: n-dotierter Halbleiter - intrisisch halbleitfähige Schicht - p-dotierter Halbleiter; oder
p-n oder n-p-Übergänge.

## Claims

1. Use of chinoid bisimidazoles and derivatives thereof as p-dopant for doping an organic semiconducting matrix material in electronic or optoelectronic structural elements, wherein the chinoid bisimidazoles are represented by the following formula: 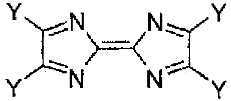 wherein each Y is independently selected from the group consisting of hydrogen, substituted and unsubstituted arylene, substituted and unsubstituted heteroarylene, substituted and unsubstituted conjugated hydrocarbon chains having altenating C-C single and double bonds, halogen, cyano, pseudohalogen, nitro, fluoro- and perfluoroalkylene, carboxylic acid and derivatives thereof, sulfonic acids and derivatives thereof, or wherein two adjacent Y are element of an aromatic ring system annelated to the imidazole ring.

2. Use according to claim 1, **characterized in that** an annelated aromatic ring system is substituted to the imidazole ring.

3. Organic semiconducting material comprising at least one organic matrix compound and a p-dopant, **characterized in that** the p-dopant is at least one compound having the following formula:

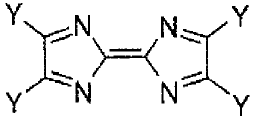

4. Organic semiconducting material according to claim 3, **characterized in that** an annelated aromatic ring system is substituted to the imidazole ring.

5. Organic semiconducting material according to claim 3 or 4, **characterized in that** the molar doping ratio of p-dopant to matrix molecule, respectively the doping ratio of p-dopant to monomeric unit of a polymeric matrix molecule is between 20:11 and 1:100.000, preferably 10:1 and 1:1.000, particularly preferred 1:1 and 1:100.

6. Electronic or optoelectronic structural element with an electronically functional area, **characterized in that** the electronically functional area has an organic semiconducting matrix material, having at least one p-dopant to modify the electronic properties of the semiconducting matrix materials by using at least one compound having the following formula: 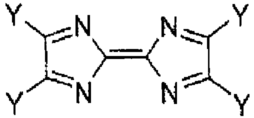 wherein each Y is independently selected from the group consisting of hydrogen, substituted and unsubstituted arylene, substituted and unsubstituted heteroarylene, substituted and unsubstituted conjugated hydrocarbon chains having altenating C-C single and double bonds, halogen, cyano, pseudohalogen, nitro, fluoro- and perfluoroalkylene, carboxylic acid and derivatives thereof, sulfonic acids and derivatives thereof, or wherein two adjacent Y are element of an aromatic ring system annelated to the imidazole ring.

7. Electronic or optoelectronic structural element according to claim 6, **characterized in that** an annelated aromatic ring system is substituted to the imidazole ring.

8. Electronic or optoelectronic structural element according to claim 6 or 7 in the form of an organic light-emitting diode, a photovoltaic cell, an organic solar cell, an organic diode or an organic field effect transistor.

9. Use of chinoid bisimidazoles and derivatives thereof according to claim 1 as p-dopant in a layer, wherein in the electronic or optoelectronic structural element this p-doped layer is present on basis of an organic matrix material in one of the following layer structures:
p-i-n: with p-dopant p-doped semiconductor - intrinsic semiconducting layer - n-doped semiconductor;
n-i-p: n-doped semiconductor - intrinsic semiconducting layer - p-doped semiconductor; or
p-n or n-p junctions.

## Revendications

1. Utilisation de bisimidazoles quinoïdes et de leurs dérivés en tant que dopant p destiné au dopage d'une matière matricielle organique semi-conductrice dans des composants électroniques ou optoélectroniques, les bisimidazoles quinoïdes étant représentés par la formule suivante : 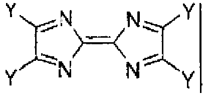 dans laquelle chaque Y est choisi indépendamment dans le groupe comprenant l'hydrogène, les aryles substitués et non substitués, les hétéroaryles substitués et non substitués, les chaînes hydrocarbures conjuguées, substituées et non substituées avec des liaisons simples et des doubles liaisons C-C alternées, l'halogène, le cyano, le pseudohalogène, le nitro, le fluoroalkylène et le perfluoroalkylène, l'acide carboxylique et ses dérivés, les acides sulfoniques et leurs dérivés, ou dans laquelle deux Y voisins sont un élément d'un système cyclique aromatique condensé sur le cycle imidazole.

2. Utilisation selon la revendication 1, **caractérisé en ce que** le système cyclique aromatique condensé sur le cycle imidazole est substitué.

3. Matière organique semi-conductrice, contenant au moins un composé matriciel organique et un dopant p, **caractérisée en ce qu'**on utilise en tant que dopant p au moins un composé de la formule suivante : 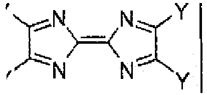 dans laquelle chaque Y est choisi indépendamment dans le groupe comprenant l'hydrogène, les aryles substitués et non substitués, les hétéroaryles substitués et non substitués, les chaînes hydrocarbures conjuguées, substituées et non substituées avec des liaisons simples et des doubles liaisons C-C alternées, l'halogène, le cyano, le pseudohalogène, le nitro, le fluoroalkylène et le perfluoroalkylène, l'acide carboxylique et ses dérivés, les acides sulfoniques et leurs dérivés, ou dans laquelle deux Y voisins sont un élément d'un système cyclique aromatique condensé sur le cycle imidazole.

4. Matière organique semi-conductrice selon la revendication 3, **caractérisée en ce que** le système cyclique aromatique condensé sur le cycle imidazole est substitué.

5. Matière organique semi-conductrice selon la revendication 3 ou 4, **caractérisée en ce que** le rapport de dopage molaire du dopant p à la molécule de la matrice ou le rapport de dopage du dopant p à des unités monomères d'une molécule polymère de la matrice est de 20 : 1 à 1 : 100.000, de préférence de 10 : 1 à 1 : 1000, de manière particulièrement préférentielle, de 1 : 1 à 1 : 100.

6. Composant électronique ou optoélectronique avec une zone fonctionnelle active du point de vue électronique, **caractérisé en ce que** la zone fonctionnelle active du point de vue électronique comporte une matière matricielle organique semi-conductrice, laquelle est dopée avec au moins un dopant p pour la modification des propriétés électroniques de la matière matricielle semi-conductrice, en utilisant un composé de la formule suivante : 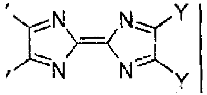 dans laquelle chaque Y est choisi indépendamment dans le groupe comprenant l'hydrogène, les aryles substitués et non substitués, les hétéroaryles substitués et non substitués, les chaînes hydrocarbures conjuguées, substituées et non substituées avec des liaisons simples et des doubles liaisons C-C alternées, l'halogène, le cyano, le pseudohalogène, le nitro, le fluoroalkylène et le perfluoroalkylène, l'acide carboxylique et ses dérivés, les acides sulfoniques et leurs dérivés, ou dans laquelle deux Y voisins sont un élément d'un système cyclique aromatique condensé sur le cycle imidazole.

7. Composant électronique ou optoélectronique selon la revendication 6, **caractérisé en ce que** le système cyclique aromatique condensé sur le cycle imidazole est substitué.

8. Composant électronique ou optoélectronique selon la revendication 6 ou 7 sous la forme d'une diode électroluminescente organique, d'une cellule photovoltaïque, d'une cellule solaire organique, d'une diode organique ou d'un transistor organique à effet de champ.

9. Utilisation de bisimidazoles quinoïdes et de leurs dérivés selon la revendication 1, en tant que dopant p dans une couche, sachant que ladite couche dopée p est présente dans le composant électronique ou optoélectronique sur la base d'une matière matricielle organique selon les structures en couches suivantes :
p-i-n : semi-conducteur dopé p avec un dopant p - couche intrinsèquement semi-conductrice - semi-conducteur dopé n ;
n-i-p : semi-conducteur dopé n - couche intrinsèquement semi-conductrice - semi-conducteur dopé p ; ou
jonctions p-n ou n-p.
